# EUROPEAN PATENT SPECIFICATION

(11) **EP 3 716 852 B1**
(45) Date of publication and mention of the grant of the patent: **02.11.2022**
(21) Application number: 18822444.8
(22) Date of filing: 07.12.2018
(51) Int. Cl.: A61B 6/00, H01L 27/146, G01T 1/29

(54) **DIRECT DETECTION AND IMAGING OF CHARGED PARTICLES FROM A RADIOPHARMACEUTICAL**
DIREKTE DETEKTION UND BILDGEBUNG GELADENER TEILCHEN AUS EINEM RADIOPHARMAKON
DÉTECTION DIRECTE ET IMAGERIE DE PARTICULES CHARGÉES À PARTIR D'UN PRODUIT RADIOPHARMACEUTIQUE

(30) Priority: 15.12.2017 GB 201721044
(43) Date of publication of application: 07.10.2020
(73) Proprietor: Lightpoint Medical Ltd, Chesham HP5 1PE (GB)
(72) Inventor: TUCH, David, Waterside, Chesham HP5 1PE (GB); VYAS, Kunal, Waterside, Chesham HP5 1PE (GB)
(74) Representative: HGF
(86) International application number: PCT/GB2018/053565
(87) International publication number: WO 2019/116010

(56) References cited:
- WO-A1-2017/212977
- JP-A- 2005 241 334
- US-A- 5 932 879
- US-A1- 2002 196 898
- US-A1- 2004 251 421
- US-A1- 2009 004 768
- US-A1- 2009 057 562
- US-A1- 2010 264 319
- US-A1- 2016 097 863
- US-A1- 2017 343 460

## Description

### TECHNICAL FIELD

The present disclosure relates to the detection of charged particles. In particular, the present disclosure relates to apparatuses, devices, methods and systems for in vivo or ex vivo detection and/or imaging of charged particles, such as electrons or positrons, emitted from a radiopharmaceutical.

### BACKGROUND

Radiopharmaceuticals, also known as medicinal radiocompounds, are a group of radioactive pharmaceutical drugs, that are often used as diagnostic and therapeutic agents. For some diagnostic or clinical reasons, such as for radioguided surgery, a radiopharmaceutical may be administered to a subject ahead of a procedure, the radiopharmaceutical designed to locate abnormal tissue such as tumours in the subject and to emit radiation detectable by specialised detection apparatus. In some circumstances, a tissue sample may be excised from the subject during surgery and the tissue sample may be analysed for radiation in order to make a determination, for example, as to whether or not the entirety of a tumour has been excised from the subject. In other circumstances, such as in keyhole or laparoscopic surgery, a laparoscopic probe is inserted through a trocar into an incision in the body of the subject and specialised detection apparatus in a probe head of the laparoscopic probe may be used to guide a surgeon towards the abnormal tissue in the subject, thereafter to treat/excise the abnormal tissue.

Many radiopharmaceuticals emit radiation in the form of charge carriers, for example in the form of electrons or positrons. For example, many radiopharmaceuticals emit beta radiation. Detection or imaging of charged particles from a radiopharmaceutical is conventionally achieved using a scintillator and a photodetector. A scintillator is a material that exhibits scintillation (luminescence) in response to ionizing radiation; when struck by a charged particle, the scintillator absorbs the energy of the charged particle and re-emits energy in the form of light. Conventional detection devices for charged particles from radiopharmaceuticals operate using the photodetector to detect the light emitted from the scintillator in response to received radiation.

However, this conventional approach to imaging charged particles from a radiopharmaceutical using a scintillator and a photodetector has a low efficiency and a low specificity for the particle of interest. For example, gamma particles may also cause a scintillator to scintillate. Additionally, this conventional approach requires engineering the interfaces between the scintillator and the photodetector which adds to signal losses. Furthermore, additional scattering processes of the photons within the scintillator leads to a loss of resolution.

The present disclosure provides solutions to address problems such as those described above.

Documents US5932879 A and US2016097863 A1 may each be considered to disclose a laparoscopic probe for detecting radiation from a radiopharmaceutical administered to a subject, the laparoscopic probe comprising a detection device, the detection device comprising: a radiation sensor having a plurality of components providing a pixel array; and a light sealing covering.

### SUMMARY

According to an aspect of the invention, a detection device is provided, the detection device for detecting radiation from a radiopharmaceutical administered to a subject. The detection device comprises a radiation sensor having a plurality of metal-oxide-semiconductor (MOS) components providing a pixel array. A semiconductor of the MOS components is configured for interaction charge carriers to be created in the depletion layer of the semiconductor in response to direct interaction with received charged particles emitted from the radiopharmaceutical. The detection device further comprises a light sealing covering arranged to prevent light from impinging on the pixel array.

A radiation sensor as described herein is a sensor able to detect ionizing particles (such as, for example, electrons or positrons) and is able to provide the user with an indication of one or more properties of said particles, such as the energy, the intensity or the location of the source of the radiation. A radiation sensor may provide information such as the presence or absence of radiation.

Using a radiation sensor such as that described herein for detecting charged particles from a radiopharmaceutical advantageously allows for greater specificity for the particle of interest than using a conventional scintillator and photodetector combination to detect charged particles. Some radiopharmaceuticals emit low levels of gamma radiation as well as beta radiation, and positron annihilation in the human body may also lead to gamma radiation noise. However, gamma radiation of the wavelengths associated with such noise is likely to pass through the radiation sensor largely without interaction with the radiation sensor, and so background noise captured in any measurement is reduced. Furthermore, even when gamma radiation does interact with the radiation sensor, post-processing may be applied to the signal(s) from the pixel array in order to largely filter out noise from the gamma radiation.

The depletion depth of the semiconductor(s) effectively determines the thickness of the active part of the radiation sensor. The thicker the depletion depth, the longer the potential interaction between the charged particle and sensor. A radiation sensor may be selected for a particular radioisotope of interest, for example by selecting a radiation sensor having a depletion depth similar to or approximately equal to the interaction depth of the emitted particle species.

The detection device may comprise a collimator, for example a parallel-hole collimator. The collimator may be arranged adjacent to the pixel array. The collimator may be to filter out charged particles that impinge upon the collimator at an angle above a threshold angle of incidence. The collimator may thereby cooperate with the light sealing covering and the radiation sensor to enable detection by the radiation sensor of a radiation imaging effect. The collimator may be formed from plastic.

By providing a collimator such as a parallel-hole collimator, when using the detection device, one is better able to determine the direction of the source of the radiation. For example, when analysing a tissue sample having abnormal tissue distributed therein, a radiopharmaceutical bound to at least some of the abnormal tissue, the parallel-hole collimator may filter out charged particles impinging on the collimator unless those charged particles approach from a suitable direction. In this way, the radiation sensor of the detection device is enabled to detect a radiation imaging effect, and so an operator of the detection device may be able to image the distribution of the radiopharmaceutical within the tissue sample.

The radiation sensor may comprise an image sensor. For example, the radiation sensor of the detection device may comprise a complementary metal-oxide-semiconductor (CMOS) image sensor / camera, which is arranged to detect charged particles but not to detect light (by virtue of at least the light sealing covering). The CMOS camera may be adapted in order to be able to detect the charged particles, for example by removing any borosilicate windows etc. of the CMOS camera that would prevent beta radiation from being sensed. The image sensor may comprise a photovoltaic cell, such as a Cadmium telluride (CdTe) photovoltaic cell.

The detection device may be operable in a first mode, in which the detection device is configured to enable detection by the radiation sensor of a radiation imaging effect. The detection device may further be operable in a second mode, in which the detection device is configured to enable detection by the radiation sensor of the presence of charged particles. For example, in the first mode, the detection device may be operable to determine the location of sources of radiation, from which a determination of, for example, a shape and size of a tumour may be interpreted; in the second mode the detection device may be configured to determine the presence or absence of charged particles in order to guide an operator of the detection device towards a radiation source (for example, during laparoscopic surgery), possibly prior to the detection device being operated in the first mode for imaging. The detection device may comprise switching means, such as a mechanical or digital switch, for switching between the first mode and the second mode (and/or vice versa).

The charged particles from the radiopharmaceutical may comprise electrons or positrons. For example, the radiation emitted from the radiopharmaceutical may comprise beta radiation.

The pixels of the pixel array may be of a size such that, in response to interaction with received charged particles from the radiopharmaceutical, the created interaction charge carriers in the depletion layer are detectable across multiple pixels. Advantageously, by providing such a pixel array, one is better able to distinguish between the detection of one or more charged particles and noise such as, for example, gamma radiation.

The detection device may further comprise a gamma radiation detector configured to detect gamma radiation. The detection device may be configured to be switchable between a first mode, in which the detection device is configured to detect charged particles (such as beta radiation), and a second mode, in which the detection device is configured to use the gamma radiation detector to detect gamma radiation. The gamma radiation detector may comprise a scintillator configured to scintillate in response to received gamma radiation. The gamma radiation detector may further comprise a photodetector to detect the scintillated light from the scintillator. The photodetector may comprise a silicon photomultiplier (SiPM). The photodetector may comprise an avalanche photodiode (APD).

A detection device having both a radiation sensor as described above and a separate gamma radiation detector can be used to detect both beta radiation and gamma radiation. Additionally, knowledge acquired using the gamma radiation detector can be used to correct for some noise in information received from the radiation sensor.

The detection device may further comprise a biocompatible shield for contact with the subject. Additionally, or alternatively, the light sealing covering may comprise a biocompatible shield for contact with the subject. A biocompatible shield may comprise, for example, an aluminium coated polyester film. A biocompatible shield allows for safe contact with the subject. A biocompatible shield may be made from, for example, a thin film of plastic resin such as Polytetrafluoroethylene (PTFE) or Polyethylene Terephthalate (PET).

The detection device may further comprise a stand-off for separating the radiation sensor from the subject by a predetermined distance. The stand-off may be for example an abutment, a guard, a barrier, a contact, a buffer or a pad, but is not limited to these examples. The stand-off may be arranged such that the radiation sensor is always held at least a minimum distance from the subject, in order for imaging of tissue to be improved.

The detection device may further comprise communication means for communicating detection by the radiation sensor of charged particles to a computing device, the computing device for processing the communicated detection and signalling the detection to a user. The communication means may comprise, for example, an optical fibre but may take any suitable form. For example, the communication means may comprise a transmitter for transmitting a communication to a receiver of the computing device.

The detection device may comprise a computing device comprising a processor for processing detection events and for signalling a detection to a user. The processor may further be configured to distinguish detection events resulting from charged particles from detection events resulting from gamma radiation. To distinguish detection events resulting from charged particles from detection events resulting from gamma radiation, the processor may be configured to receive a signal from the radiation sensor, the signal representative of interaction charge carriers being created in the depletion layer of the semiconductor of the plurality of MOS components providing a pixel array. The processor may further be configured to determine whether the signal is indicative of detection events at multiple neighbouring pixels of the pixel array. The processor may further be configured to, if a determination is made that the signal is indicative of detection events at multiple neighbouring pixels of the pixel array, determine that the radiation sensor has received at least one charged particle. The processor may further be configured to, if a determination is made that that signal is not indicative of detection events at multiple neighbouring pixels of the pixel array, determine that the radiation sensor has received gamma radiation. In some embodiments, the processor may further be configured to discard detection events resulting from gamma radiation.

Advantageously, by providing the computing device as part of the detection device, all processing may be performed by the detection device without the need for further computing devices.

The radiation sensor may be selected such that the semiconductor of the MOS components has an optimal depletion layer depth for an energy spectrum of charged particles particular to the radiopharmaceutical. In this way, the specificity of the detection device for detecting a particular radiopharmaceutical is improved. For example, the depletion layer depth may be between three and five nm.

The detection device may be a handheld detection device. For example, a handheld detection device may be suitable for a medical professional to efficiently obtain a reading by holding the device at or near a subject's skin.

According to an aspect of the invention, a laparoscopic probe is provided, the laparoscopic probe comprising a detection device as described herein. Such a laparoscopic probe may be used to perform keyhole surgery on a subject that has been administered with a beta-emitting radiopharmaceutical and, due to the benefits described above of using such a detection device, the laparoscopic scope is better suited for such radioguided laparoscopic surgery than a laparoscopic probe that uses a conventional scintillator and photodetector for detecting beta radiation.

The laparoscopic probe may be modular. For example, the detection device of the laparoscopic probe may be interchangeable with a second detection device. The field of view of the radiation sensor of the detection device may be different from the field of view of the radiation sensor of the second detection device. In this way, such a modular laparoscopic probe may be used to obtain multiple, varied views.

The laparoscopic probe may comprise a grip for manipulating the laparoscopic probe inside the cavity with a surgical tool. The grip may be bevelled, in order to be more easily gripped by a surgical tool. The grip may be magnetic, in order to be more easily gripped by a surgical tool.

According to an aspect of the invention, a specimen imaging apparatus is provided. The specimen imaging apparatus comprises a detection device as described herein. The specimen imaging apparatus further comprises a light tight enclosure within which a tissue sample can be received at a sample location, the tissue sample excised from the subject subsequent to the radiopharmaceutical being administered to the subject.

Conventionally, when excising a tumour or other tissue abnormality, a surgeon may need to decide on an amount of tissue containing the tumour to remove, with the aim of removal of the entirety of the tumour. However, when deciding how much tissue should be removed, there is a trade-off between removing as little tissue as possible to attempt to encompass the tumour without going beyond the margin of the tumour and into healthy tissue, and removing more than is necessary to ensure that the entire tumour has been removed from the patient yet causing collateral damage. Removing too much tissue can cause adverse post-operative effects for a patient. During surgery, a surgeon will conventionally make a judgement call based on experience and a tactile assessment of the excised sample to decide whether sufficient tissue has been removed to capture all of the tumour. If a surgeon is satisfied that the tissue excised is sufficient, the surgeon will close the incision, end the surgery, and send the patient home for recovery while the tissue sample is sent to a pathology laboratory for histological analysis. Frequently, it is discovered that the abnormal tissue or tumour is broaching the surface or is too close to the surface of the excised tissue sample to be confident that the entire abnormal tissue/tumour has been removed. That is, the histological analysis suggests that abnormal or cancerous tissue may have been left inside the patient, or the margins of tumour-free tissue towards the exterior of the tissue sample are too small to guarantee that all of the abnormal tissue or tumour has been removed from a patient. The patient may need to be recalled for reoperation in order to remove further tissue, which can be worrisome and unpleasant for the patient and requires further time and labour resources to be expended.

However, a specimen imaging apparatus as described herein may be used to obtain quick results during surgery. As beta radiation decays away over a distance of around 1mm in tissue, if an excised sample is supplied to such a specimen imaging apparatus during surgery, the detection device of the specimen imaging apparatus may be used to indicate whether or not the abnormal tissue is broaching the surface of the excised sample; that is, to better determine whether to send the patient home to recover.

The specimen imaging apparatus may further comprise at least a second radiation sensor having a different field of view to the field of view of the radiation sensor. In this way, multiple fields of view of the sample may be captured at the same time. The second radiation sensor may also comprise a plurality of metal-oxide-semiconductor (MOS) components providing a pixel array, a semiconductor of the MOS components configured for interaction charge carriers to be created in the depletion layer of the semiconductor in response to direct interaction with received charged particles emitted from the radiopharmaceutical. For example, the second radiation sensor may comprise a CMOS image sensor. The specimen imaging apparatus may also comprise a further light sealing covering for preventing light from reaching the second radiation sensor.

The specimen imaging apparatus may further comprise an illumination source. The specimen imaging apparatus may further comprise a camera for capturing a white light image of a sample provided to the light tight enclosure when the sample is illuminated, for example, by the illumination source. The specimen imaging apparatus may further comprise a visual display configured to display a white light image of the sample and an image representative of charged particles from a radiopharmaceutical in the sample. By providing an illumination source and a camera, a white light image of the sample may be captured. Optionally, a radiation image effect captured by the radiation sensor of the detection device may be overlaid upon a white light image of the sample (or vice versa) in order to provide a visual indication as to where abnormal tissue, if visible to the radiation sensor, is located within the sample.

According to an aspect of the invention, a method of using a detection device as described herein is provided. The method comprises receiving a detection signal from the radiation sensor of the detection device, the detection signal representative of interaction charge carriers being created in the depletion layer of the semiconductor of the plurality MOS components providing a pixel array. The method further comprises determining whether the detection signal is indicative of detection events at multiple neighbouring pixels of the pixel array. The method further comprises, if a determination is made that the detection signal is indicative of detection events at multiple neighbouring pixels of the pixel array, determining that the radiation sensor has received at least one or more charged particles. Advantageously, such a method allows one to distinguish detections of charged particles from other noise.

The radiation sensor may comprise an image sensor. Receiving a detection signal from the radiation sensor may comprise receiving image data from the image sensor, the image data representative of a radiation imaging effect. Determining whether the detection signal is indicative of detection events at multiple neighbouring pixels of the pixel array may comprise comparing the received image data with fixed pattern noise data to produce a corrected image, the fixed pattern noise data derived from an average of a plurality of dark noise images collected using the detection device. Determining whether the detection signal is indicative of detection events at multiple neighbouring pixels of the pixel array may further comprise comparing pixel values of pixels of the corrected image with a threshold value to produce a binary image, wherein the pixel value of each pixel of the binary image is representative of whether the pixel value of a corresponding pixel of the corrected image is above the threshold value. Determining whether the detection signal is indicative of detection events at multiple neighbouring pixels of the pixel array may further comprise, for at least one pixel of the binary image, determining how many adjacent pixels have the same value as the pixel.

The method may further comprise, if a determination is made that that signal is not indicative of detection events at multiple neighbouring pixels of the pixel array, determining that the radiation sensor has received gamma radiation.

The method may further comprise operating the detection device in a first mode, in which the detection device is configured to enable detection by the radiation sensor of a radiation imaging effect; and operating the detection device in a second mode, in which the detection device is configured to enable detection by the radiation sensor of the presence of charged particles.

According to an aspect of the invention, a computer-readable medium is provided, the computer-readable medium having executable instructions stored thereon which, when executed by a processor, cause a method as described herein to be performed / carried out. The computer-readable medium may comprise a non-transitory computer-readable medium. The computer program and/or the code for performing such methods as described herein may be provided to an apparatus, such as a computer or other computing device, on the computer-readable medium or other computer program product. The computer-readable medium could be, for example, an electronic, magnetic, optical, electromagnetic, infrared, or semiconductor system, or a propagation medium for data transmission, for example for downloading the code over the Internet. Alternatively, the computer-readable medium could take the form of a physical computer-readable medium such as semiconductor or solid-state memory, magnetic tape, a removable computer diskette, a random access memory (RAM), a read-only memory (ROM), a rigid magnetic disc, and an optical disk, such as a CD-ROM, CD-R/W or DVD

According to an aspect of the invention a computing device is provided. The computing device comprises a memory, the memory having instructions stored thereon which, when executed by a processor, cause a method as described herein to be performed / carried out. The computing device may further comprise a processor configured to execute the instructions stored in the memory.

Many modifications and other embodiments of the inventions set out herein will come to mind to a person skilled in the art to which these inventions pertain in light of the teachings presented herein. Therefore, it will be understood that the disclosure herein is not to be limited to the specific embodiments disclosed herein. Moreover, although the description provided herein provides example embodiments in the context of certain combinations of elements, steps and/or functions may be provided by alternative embodiments without departing from the scope of the invention.

### BRIEF DESCRIPTION OF THE DRAWINGS

Embodiments of the invention are further described hereinafter with reference to the accompanying drawings, in which:
Figure 1A is an illustration of a radiation sensor;
Figure 1B shows circuitry of the radiation sensor of Figure 1A;
Figure 1C shows circuitry of a pixel of the radiation sensor of Figure 1A;
Figure ID shows a p-n junction;
Figure 2 is a schematic of a laparoscopic probe comprising a detection device as described herein;
Figure 3 is a schematic of a specimen imaging apparatus comprising a detection device as described herein;
Figure 4 is a flowchart of a method of operating a detection device as described herein; and
Figure 5 is a block diagram of a computing device.

Throughout the description and drawings, like reference numerals refer to like parts.

### DETAILED DESCRIPTION

The present disclosure seeks to provide improved methods, systems, apparatuses and devices for detecting radiation in the form of charged particles emitted from a radiopharmaceutical administered to a subject. Whilst various embodiments are described below, the invention is not limited to these embodiments, and variations of these embodiments may well fall within the scope of the invention which is to be limited only by the appended claims.

As explained above, a conventional detection device for detecting radiation from a radiopharmaceutical administered to a subject comprises a scintillator, the scintillator for absorbing the energy of received radiation and emitting light, and a photodetector for detecting the scintillated light and generating an electrical signal to indicate the detection. However, as indicated above, there are a number of difficulties in designing and using such conventional detection devices.

As will be discussed below, the inventors have developed detection devices for detecting radiation, specifically charged particles such as electrons or positrons, from a radiopharmaceutical administered to a subject. These detection devices utilise solid state radiation sensors, and specifically semiconductor solid state radiation sensors.

A radiopharmaceutical is a drug that can be used for diagnostic or therapeutic purposes and comprises a radioisotope bonded to a molecule. The radiopharmaceutical conveys the isotope to specific organs, tissues or cells. The radiopharmaceutical is selected for its properties and purpose. Many radiopharmaceuticals are known in the art and are used for radioguided surgery and other procedures. The radionuclides can usually be categorised by their decay modes, namely alpha decay, beta decay (electrons or positrons), electron capture and/or isomeric transition. Some beta decaying radioisotopes, including Fluorine-18 (¹⁸F), Carbon-11 (¹¹C), Nitrogen-13 (¹³N), Copper-64 (⁶⁴Cu), Iodine-124 (¹²⁴I) and Gallium-68 (⁶⁸Ga), emit positrons during radioactive decay and are known to be used in positron emission tomography (PET) imaging. Some beta decaying radioisotopes, including tritium (³H), Carbon-14 (¹⁴C), and Silicon-35 (³⁵S), emit electrons during radioactive decay. Some radiopharmaceuticals are primarily gamma radiation emitters but may also emit charged particles. For example, Technetium-99m (^{99m}Tc) is a gamma radiation emitter, but through a process of internal conversion emits K, L or M shell electrons for approximately 10% of decays.

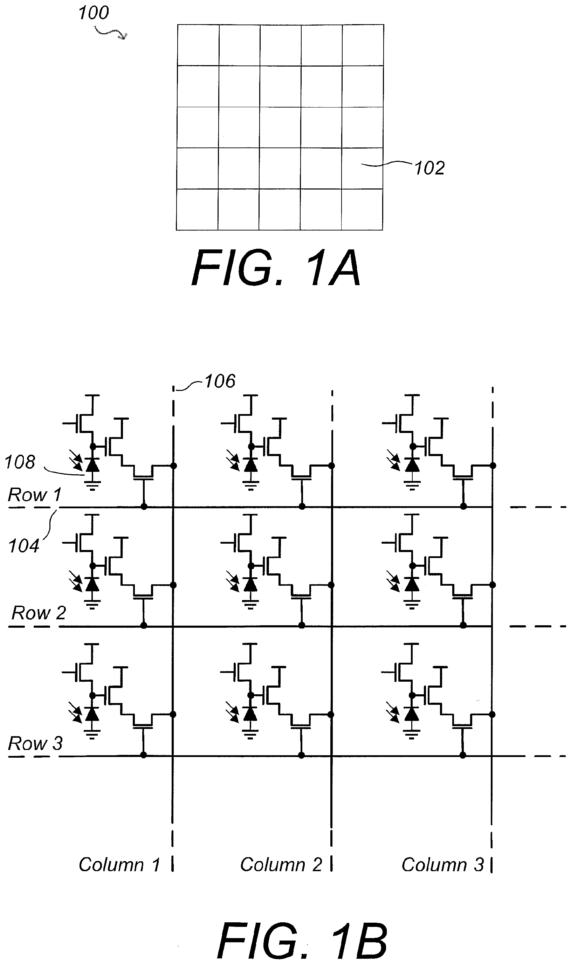

In embodiments, the radiation sensor may comprise a complementary metal-oxide-semiconductor (CMOS) image sensor, for example a CMOS Active Pixel Sensor which will briefly be described with reference to Figures 1A to ID. The skilled person would understand that although a CMOS Active Pixel Sensor is described herein, this is just one way of implementing the detection devices described herein, and other architectures and designs are possible. Other solid-state semiconductor radiation sensors may additionally or alternatively be utilised, for example, radiation sensors comprising charge coupled devices (CCDs), or silicon drift detectors (SDDs), PIN photodiodes etc.

Figure 1A illustrates a radiation sensor 100, which in the present example comprises a CMOS radiation sensor. The radiation sensor 100 comprises a plurality of metal-oxide-semiconductor (MOS) components which provide a pixel array comprising a plurality of pixels 102. In Figure 1A, the pixel array comprises 8 pixels in each row and 8 pixels in each column, although the skilled person would appreciate that more of fewer pixels 102 may form such a pixel array.

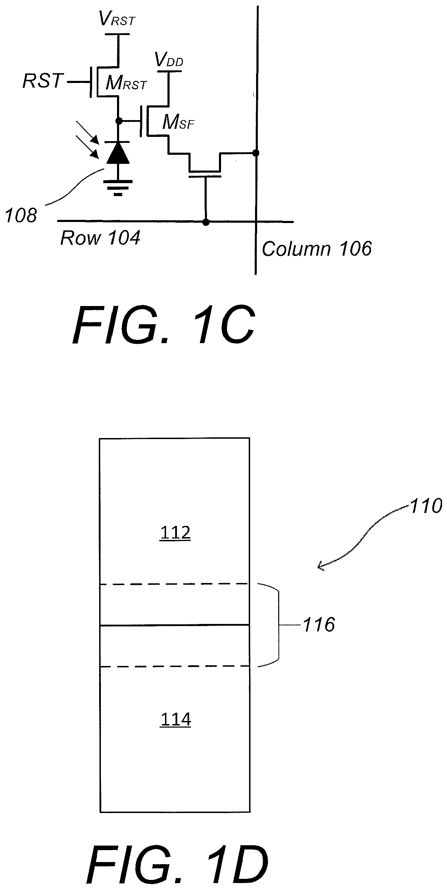

An example architecture of CMOS radiation sensor 100 is shown in Figure 1B, and Figure 1C shows the electronics of a pixel 102 of the sensor 100 (although the skilled person would appreciate that other designs may be used). In Figure 1B only 9 pixels are shown (3 pixels in each row and 3 pixels in each column), although the skilled person would appreciate that the pixel array may comprise more or fewer pixels. Each pixel 102 comprises a photodiode 108 (see Figure ID), which comprises at least a p-n junction 110. A p-n junction 110 comprises a boundary or interface between a p-type semiconductor 112 and an n-type semiconductor 114. The p-type semiconductor 112 has an excess of holes (positively charged quasiparticles) and the n-type semiconductor 114 has an excess of electrons. At the junction, a depletion layer/region 116 is formed. When a charged particle with an energy at or near the band gap crosses the depletion region 116 of the p-n junction 110, interaction charge carriers (electron-hole pairs) are created in the depletion layer 116, separated by the electric field of the junction, and collected at electrodes of the photodiode 108. The MOS components of the radiation sensor 100 may have a depletion layer 116 selected for specificity to an energy of emitted charge carriers from the radiopharmaceutical of interest, as will be explained further below.

With reference to Figure 1C, the photodiode 108 is connected to a reset voltage V_{RST} through a transistor M_{RST}. A reset controller (not shown) may apply a current to open and close the channel in the transistor M_{RST} via gate RST, thereby ensuring that the voltage at the end of the photodiode 108 is V_{RST}. If the photodiode 108 is exposed to radiation, then the channel of a read-out transistor M_{SF} is affected. The read-out transistor M_{SF} is connected to a voltage source for amplification V_{DD}. After a predetermined integration time (in which the photodiode 108 may or may not detect radiation from a radiopharmaceutical), a row controller (not shown) applies a current to row 104 and the current on column 106 is detected. The resistance of M_{SF} (which reflects whether or not the photodiode 108 received ionizing radiation) has an effect on the measured current of column 106. In this way, a radiation sensor 100 may operate.

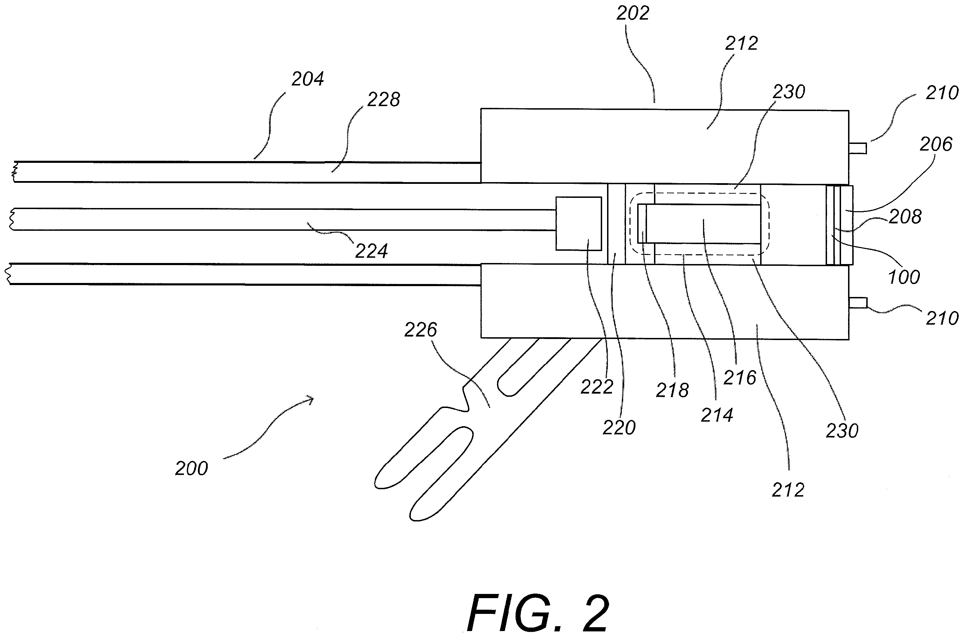

Figure 2 is a schematic of a laparoscopic probe 200 comprising a probe head 202 and a connection portion 204 for reporting to a computing device (such as computing device 500 described in detail below). In the particular example shown in Figure 2, the laparoscopic probe comprises a tethered laparoscopic probe, in which the probe head is designed for insertion through a trocar into a cavity in the subject under examination (e.g. a patient) and to be freely moveable inside the cavity. The connecting portion 204 of the laparoscopic probe therefore additionally acts to, in use, tether the probe head through the trocar (not shown). To this end the laparoscopic probe 200 also comprises a deployable/retractable grip 226 for manipulating the probe head 202 of the laparoscopic probe 200 in use with a surgical tool. The grip may be bevelled for improved grip, and/or may be magnetic.

The connecting portion 204 may comprise a biocompatible casing 228 and one or more optical fibres 224 for communicating to the computing device (not shown). The skilled person would appreciate that other laparoscopic probe architectures may be used, for example in which the connecting portion 204 is a rigid, rod-like structure.

The probe head comprises an outer casing 212. The outer casing 212 is biocompatible.

Referring to the figure, a radiation sensor 100 is located towards a detection end of the probe head 202. The radiation sensor 100 may comprise a CMOS image sensor such as that described above in relation to Figures 1A-1D.

A thin light sealing covering 208 is provided in front of the radiation sensor 100 (that is, located between the radiation sensor 100 and the detection end of the probe head 202). The light sealing covering 208 is substantially opaque to light and is arranged such that, in use, the light sealing covering prevents light from impinging on the pixel array of the radiation sensor 100 and so the number of false positive detection events by the radiation sensor 100 is greatly reduced. The light sealing covering 208 of laparoscopic probe 200 is permeable to charged particles from the radiation source. For example, the light sealing covering 208 may be a thin film such that beta radiation with sufficient energy can penetrate the light sealing covering 208. The outer casing 212 of the laparoscopic probe 200 shown in Figure 2 also acts to prevent light from reaching the radiation sensor 100.

In front of the light sealing covering 208 (that is, located between the light sealing covering 208 and the detection end of the probe head 202), is located a collimator 206. The collimator 206 is a parallel-hole collimator which acts to filter out radiation that impinges upon the collimator 206 at an angle above a threshold angle of incidence. This threshold angle of incidence is defined by the size of the holes of the parallel-hole collimator 206 (i.e. by the area and depth of the holes). In this way, the collimator 206 ensures that only charged particles that approach the collimator 206 at an angle that is not greater than the threshold angle of incidence can pass through the holes of the collimator 206 to be detected by the radiation sensor 100. Accordingly, the detection device in the form of laparoscopic probe 200 is able to better determine the direction of a radiation source in use.

The probe head 202 further comprises a biocompatible stand-off 210 which keeps the radiation sensor 100 at least a fixed distance away from a tissue surface in use. Accordingly, the stand-off 210 allows for the radiation sensor 100 to have better imaging capabilities.

The radiation sensor 100 is communicatively coupled to circuitry 222 (the connections between the radiation sensor 100 and the circuitry 222 are not shown in the Figure). The circuitry 222 is configured to generate a signal for transmitting along one or more optical fibres 224 to the computing device (not shown).

In the laparoscopic probe 200 shown in Figure 2 there is additionally a gamma radiation detector 214 located behind the radiation sensor 100 (that is, the radiation sensor 100 is located between the gamma radiation detector 214 and the detection end of the probe head 202). The gamma radiation detector 214 comprises a scintillator 216 and a photodetector 218 for detecting scintillated light from the scintillator 216. Upon detection, the circuitry 222 is configured to communicate detection events to the computing device (not shown).

The gamma radiation detector 214 is shielded by rear shielding 220 and side shielding 230. The rear shielding 220 and side shielding 230 may comprise tungsten. The rear shielding 220 and the side shielding 230 are arranged to inhibit gamma radiation from impinging upon the scintillator 216 through, respectively, the rear and side of the probe head 202. Accordingly, it is likely that any scintillated light from the scintillator 216 is likely to have originated from gamma radiation approaching through the detection end of the laparoscopic probe 200.

Due to the small profile of the solid-state radiation sensor 100, gamma radiation through the detection end of the laparoscopic probe 200 can pass through the radiation sensor 100 largely without interacting with pixels 102 of the radiation sensor 100. The few detection events that lead to false positives at the radiation sensor 100 (e.g. detection events resulting from gamma radiation interacting with the radiation sensor 100) can be filtered out of the signal from the radiation sensor 100 through processing at the computing device (not shown). This arrangement allows for the two different sensor types (the MOS-based radiation sensor 100 and the scintillator-based gamma radiation detector 214) to be used to monitor different types of radiation with greater specificity. The charge carrier sensor and the gamma radiation detector 214 are able to be combined into a laparoscopic probe head 202 of a size small enough to pass through a trocar largely due to the smaller profile of the radiation sensor 100.

In use, the laparoscopic probe 200 may be switchable between a first mode, in which the laparoscopic probe 200 is configured to detect charged particles (e.g. beta radiation) using the radiation sensor 100, and a second mode in which the laparoscopic probe 200 is configured to detect gamma radiation using the gamma radiation detector 214. The circuitry 222 may therefore communicate charge carrier detection events in the first mode and gamma detection events in the second mode. Alternatively, the circuitry 222 may communicate a combined signal to the computing device (not shown) which may then process the received signal.

The skilled person would understand that Figure 2 shows one non-limiting embodiment and that variations of the laparoscopic probe 200 may be permitted.

For example, a laparoscopic probe head may or may not comprise a gamma radiation detector 214, and if a gamma radiation detector is present it may take any suitable form.

The collimator 206 may or may not be present and, if present, may be positioned behind the light sealing covering 208 (e.g. such that the collimator 206 is positioned between the radiation sensor 100 and the light sealing covering 208). The collimator 206 may be made of a biocompatible material.

The stand-off 210 may or may not be present. If present the stand-off 210 may be of any suitable shape and material. Any or all of the radiation sensor 100, light sealing covering 208 and collimator 206 may be positioned further back within the outer casing 212. In this way, the outer casing 212 may itself perform the function of a stand-off.

The connection portion 204 may be flexible or may be rigid. A tethered laparoscopic probe with a flexible connection portion may be used for insertion through a trocar into a body cavity, and thereafter the probe head may have six degrees of freedom. However, the connection portion may be rigid (thereby reducing the number of degrees of freedom of the probe head in use.

In Figure 2, the radiation sensor 100 detects charged particles from the end of the laparoscopic probe 200. However, other configurations are envisaged, for example the radiation sensor 100 may be positioned to detect charged particles at the side of the probe head. In the embodiment shown in Figure 2, the gamma radiation detector 214 is also arranged to detect gamma radiation through the detection end of the laparoscopic probe 200. However, the gamma radiation detector 214 may instead be positioned at a different angle to the radiation sensor 100. For example, the radiation sensor 100 may be positioned on the side of the probe head while the gamma detector may be positioned to detect gamma radiation substantially through the detection end of the probe head, or vice versa. The data from both detectors may be processed at a computing device (not shown). The laparoscopic probe may comprise further additional radiation sensors.

The skilled person would also appreciate that the grip of grasper 226 is an optional feature.

Figure 2 thus illustrates an example of a laparoscopic probe for keyhole/laparoscopic surgery. However, the principles described herein are also applicable to detection of radiation in ex vivo tissue samples from a subject, for example when a tissue sample is excised from a subject when the subject has been administered a radiopharmaceutical ahead of the excision. Such situations may arise, for example, when a surgeon seeks to remove a tumour from a patient. In such a circumstance, a tissue sample may be excised from a subject, the subject having received a radiopharmaceutical ahead of the procedure, for example a beta-emitting radiopharmaceutical. It is known that beta radiation can usually travel a distance of around 1mm through tissue. If beta radiation can be detected from the sample S, then it is an indicator that the surgeon has not excised a large enough tissue sample to guarantee that the entire tumour has been removed.

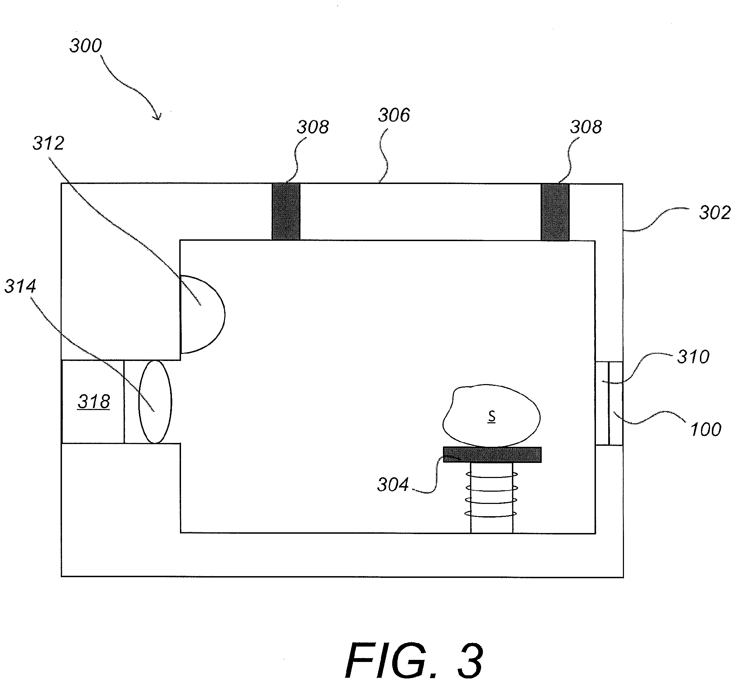

Figure 3 shows an imaging apparatus 300 which can be used to image an object, for example a tissue sample. The skilled person will appreciate that the imaging apparatus 300 may be suitable for imaging other objects. The skilled person will also comprehend that the imaging apparatus 300 of Figure 3 is described as an example only and that other architectures are available. The apparatus 300 is suitable for use by a surgeon or nurse or other medical professional in a clinical setting.

The apparatus 300 includes a light tight chamber/enclosure 302 in which a sample S can be supported on a sample platform 304. The light tight enclosure 302 may take any number of suitable forms consistent with its role of at least substantially (and preferably completely) excluding ambient light from the inside of the enclosure 302 where the sample S is received. The enclosure may be reusable.

The light tight enclosure 302 has a door 306 that can be opened to give access to the interior of the enclosure 302, for example, for introduction or removal of the sample S. The door 306 is shown in the Figure as being located at the top of the imaging apparatus 300 although the skilled person would appreciate that the door can be located elsewhere on the imaging apparatus. A seal 308 around the periphery of the door 306, for example a labyrinth seal, ensures the light tightness of the chamber 302 when the door 306 is closed. The light tight chamber 302 may be considered as a light sealing covering in that is prevents light from outside of the chamber from affecting the sensor 100 within when the imaging apparatus is in use.

The enclosure 302 and door 306 are constructed from completely opaque materials, for example 2mm thick steel sheeting. Additionally, the internal surfaces of the enclosure 302 and door 306 are preferably black with low reflectivity in order to absorb any stray light. In some embodiments, a light sensor within the enclosure 302 can be used to confirm whether or not the enclosure 302 is light tight when the door 306 is closed.

The sample platform 304 may be raised or lowered in order to alter the height of the sample S within the chamber 302. The sample platform 304 is also rotatable. The sample platform may be vertically adjustable but in some embodiments may additionally or alternatively be horizontally/laterally adjustable also. Furthermore, the angle of the sample platform 304 relative to the imaging apparatus within the chamber 302 may be adjustable. The skilled person would understand that the sample platform 304 is an optional feature - an adequate location for the sample may in some circumstances simply be the floor of the chamber 302.

A radiation detection system is mounted in the side of the apparatus 300. The radiation detection system comprises a solid-state radiation detector 100, such as the CMOS detector 100 of Figure 1, having a plurality of metal-oxide-semiconductor, MOS, components providing a pixel array, a semiconductor of the MOS components configured for interaction charge carriers to be created in the depletion layer of the semiconductor in response to direct interaction with received charged particles emitted from a radiopharmaceutical. A collimator 310 is arranged in front of the radiation sensor 100 (that is, between the radiation sensor and the sample platform 304). In some embodiments, the collimator may be configured to magnify the image of the charged particles received by the radiation sensor 100 of the sample S. In some embodiments, the collimator may be a parallel-hole collimator and may function in much the same way as described above in relation to the laparoscopic probe 200 of Figure 2. In some embodiments, there may be no collimator present. The collimator acts to improve the imaging capabilities of the radiation sensor 100.

The light tight enclosure 302 acts as a light sealing covering for the radiation sensor 100, as it functionally inhibits external light from impinging upon the radiation sensor 100 when the radiation sensor 100 is operated to detect/image charged particles from a sample. However, in some embodiments, a further light sealing cover, for example a shutter, may be provided within the light tight enclosure 302. The shutter may, for example, be arranged between the collimator 310 and the sample holder 304 and be arranged (when closed) to prevent light from within the light tight enclosure 302 from impinging upon the radiation sensor 100. The shutter 312 may be opened and closed by a computing device or controller (not shown) which may be computing device 500 described below.

Imaging apparatus 300 further comprises an illumination source 312 for illuminating the interior of the light tight enclosure 302. The light source 312 is for illuminating the interior of the enclosure 302 with white light or RGB light, which can be used to help directly image the sample S. The light source may comprise a light emitting diode (LED). The light source may comprise a combination of red, green and blue lights. The skilled person would appreciate that the light source 312 is optional. In some embodiments, light may be directed into the chamber 302 by use of, for example, optical fibres when required.

For the purpose of imaging the sample S when the sample S is illuminated by the illumination source 312, the imaging apparatus 300 may comprises one or more optical components, for example a lens 314 that is configured to pass light from within the light tight enclosure 302 to an imaging means 318 comprising a camera. In some embodiments, the lens may be outside the light tight enclosure 302, either directly in line with an aperture in the enclosure or offset from the aperture with a mirror adjacent to the aperture directing the light exiting the aperture to the lens.

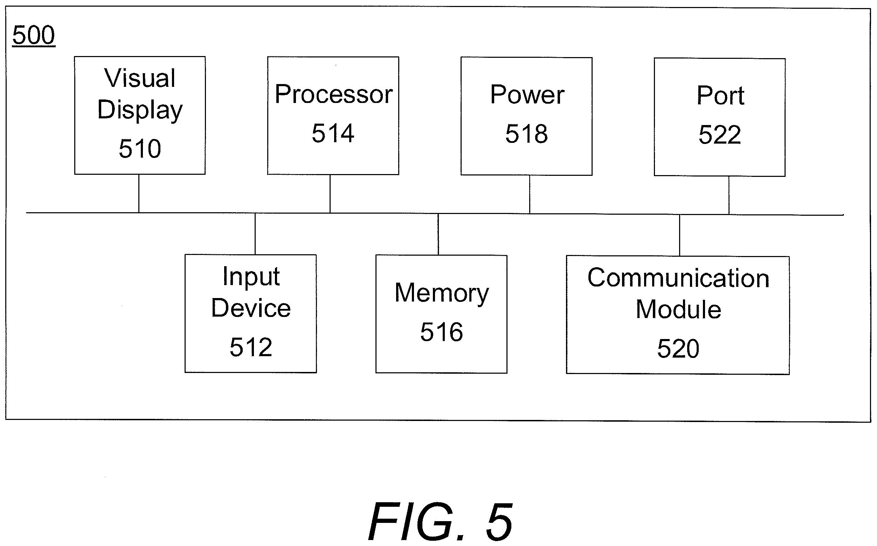

In use, a sample S may be excised from a subject, the sample S containing a radiation source from a radiopharmaceutical administered to the subject prior to the excision. The radiopharmaceutical may be, for example a beta radiation source. The excised sample S may be introduced to the light tight chamber 302 and the door 306 may be closed. The illumination source 312 may be turned off. The radiation detector 100 is exposed to receive radiation from the sample S. After a predetermined exposure time, the illumination source 312 may be turned on. An image of the sample S may be captured using the camera 318. The sample platform may be rotated, either manually or via a controller, for example the computing device 500 of Figure 5. In this way, different surfaces of the sample S are angled towards the radiation sensor 100 and towards the camera 318. Further charged particle images may be captured by the radiation sensor 100 and further white light images may be captured by the camera 318. The skilled person would appreciate that images may be captured in any order - for example a plurality of charged particle may be captured by the radiation sensor 100 prior to a plurality of white light images being captured by the camera 318. A computing device may then be able to overlay images from radiation source 100 and camera 318 in order to associate a radiation source within the sample S with an image location on the sample S. As mentioned above, as charged particles such as beta radiation cannot penetrate far through tissue, any radiation detected is likely to originate from a source within a distance of about a millimetre beneath the surface of the sample S. If radiation is detected, then an operator of the imaging apparatus 300 may determine the sample S is too small to guarantee that the entirety of the tumour has been removed from the subject.

The skilled person would appreciate that other architectures are possible. For example, the imaging apparatus does not need to include an illumination source 312 or any of the white light imaging apparatus, such as lens 314 and camera 318. The camera 318 and the radiation source may be positioned so as to image the same part of the sample S at the same time, reducing the need for post processing to match up images captured by a camera 318 with images captured by a radiation sensor 100.

The specimen imaging apparatus may comprise additional radiation sensors and/or imaging devices or cameras. For example, the specimen imaging apparatus may comprise multiple detection devices / radiation sensors, each having a different field of view and each configured to simultaneously capture an image of the tissue sample. In this way, multiple images of the sample may be captured from multiple angles at the same time and a composite image of the tissue sample may be rapidly reconstructed.

According to at least another embodiment, a detection device may be provided in the form of a pad on which a sample may be placed. The pad may comprise a very large area radiation sensor (as described herein), for example 6cm by 6cm and light sealing means such as a biocompatible light shield. The sample may be placed onto the pad such that the surface of the tissue to be imaged is face down on the pad. The tissue sample may be manipulated in order to image other sides/surfaces. In this embodiment, there is no requirement for a light tight chamber as the suspect tissue is placed into direct contact with the pad and the radiation sensor can detect any charged particles emitted from the surface of the sample.

According to at least another embodiment, a detection device may be a handheld detection device. A handheld detection device may be useful to, for example, analyse skin tissue of a subject or patient, for example to examine a mole on the skin. If a radiopharmaceutical compound binds to abnormal tissue on the skin, then an operator of the handheld detection device may be able to detect the radiopharmaceutical compound. The handheld detection device is configured to operate in much the same way as the laparoscopic probe 200 described above or the specimen imaging apparatus 300 described above. For example, a radiation sensor 100, a collimator and a light sealing covering / light shield may be integrated into a handheld device that may be communicatively coupled to a computing device such as computing device 500 described below. The skilled person would appreciate that a larger area radiation sensor may be used in a handheld detection device than in a laparoscopic probe. Furthermore, the skilled person would appreciate that the handheld detection device may comprise multiple radiation sensors in order to simultaneously determine, from multiple angular views, whether charged particles are being emitted. The handheld detection device may then be switchable between a first mode in which the device detects the presence (or absence) of charged particles, and a second mode in which the multiple radiation sensors are used for imaging. A handheld detection device may also be couplable with one or more accessories, for example a collimator. In this way, the same handheld detection device may be fitted with different accessories for different purposes.

A handheld detection device may also be used for in-situ imaging of tissue, for example during surgery.

A method of operating a detection device as described herein, may comprise receiving a detection signal from the radiation sensor of the detection device, the detection signal representative of interaction charge carriers being created in the depletion layer of the semiconductor of the plurality of MOS components providing a pixel array. The method may further comprise determining whether the detection signal is indicative of detection events at multiple neighbouring pixels of the pixel array. The method may further comprise, if a determination is made that the detection signal is indicative of detection events at multiple neighbouring pixels of the pixel array, determining that the radiation sensor has received at least one or more charged particles. The radiation sensor may comprise an image sensor such as a CMOS image sensor, and the received data from the radiation sensor may comprise image data.

In order to calibrate the detection device, it is beneficial to compare received image data with fixed pattern noise data. In order to derive the fixed pattern noise data, one may capture a plurality of dark noise images, that is, images captured in the absence of a radiopharmaceutical and light. In some embodiments, the dark noise images may be captured at the full bit depth of the radiation sensor. The number of dark noise images captured may not need to exceed 16 to remove the stochastic part of the fixed pattern noise, but larger numbers of images will account for local variations in the dark field due to power supply noise and other features which can make banding appear in images from the radiation sensor.

The plurality of dark noise images may be processed and, for example, and averaged dark image may be obtained. If the number of dark noise images is a power of 2 then the average can be deduced from a simple sum of the images, and bit shifting can be used to subsequently maintain the precision of the dark-current offset.

The averaged dark mean image is representative of fixed pattern noise of the radiation sensor, for example sensor leakage and amplifier offsets. The fixed pattern noise data may be stored using integer or floating-point arithmetic in memory of the computing device 400.

Optionally, one may also choose to dynamically update the dark current drift by using the dark signal that lies within an integer N standard deviations of the pixel mean to calculate a rolling fixed pattern noise. The value of N in such cases needs to be chosen carefully. Advantageously, by dynamically updating the averaged dark image in this way, one can compensate for any temperature dependence in the electronics of the detection device (including in the radiation sensor) or sampling in the sensor.

One may further calibrate the detection device by capturing a "bad pixel map". One may do this by capturing a number of images using the radiation sensor (e.g. 100 images at full bit depth), and shifting the image up in bit space (that is, performing a bit shift on the bits representing the pixel values) to match the number of images collected in the dark image sum (e.g. if 16 dark noise images were collected then shift the image into 14-bit space). The fixed pattern noise may then be subtracted from the collected number of images and an image offset large enough to prevent any image values from being crushed. With a 10-bit image space, this dark value is typically around 200 (i.e. ~19.5%). In 14-bit space this would be 3200. For each adapted image, it is then determined whether the pixels in that adapted image are above or below a number M times the standard deviation from the mean. If the pixel value is above or below that threshold value, then an identifier of that pixel may be added to a 1-bit pixel map and flagged as "bad". This is repeated for each image until a bad pixel map is generated.

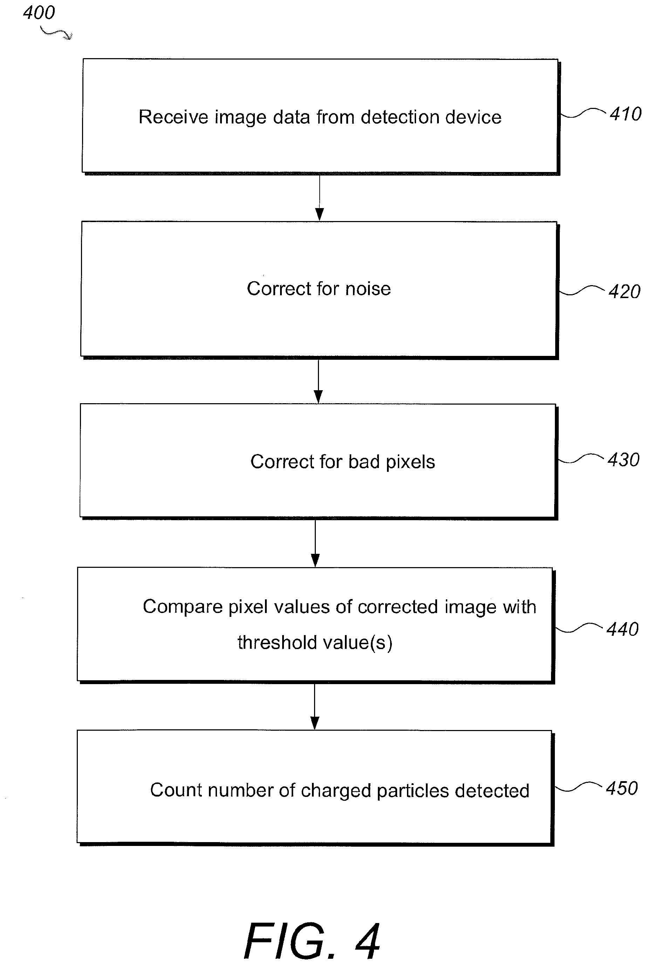

A description of a more detailed method is now described with reference to Figure 4, which shows a flowchart of a method for operating a detection device as described herein. The method may be performed by a computing device such as computing device 500 described below. The method may be performed to operate a detection device as described herein in any guise. For example, the method may be performed to process information from a laparoscopic probe such as laparoscopic probe 200, a specimen imaging apparatus such as specimen imaging apparatus 300, a handheld detection device, or any other form of the detection device.

At step 410, image data is received from the radiation sensor of the detection device. The image data may be received directly or indirectly. The image data is representative of a radiation imaging effect at an image sensor of the detection device. The image data may be received in any suitable form.

The image data may comprise an image corresponding to a single exposure of the radiation sensor. The image data may comprise one or more images corresponding to multiple exposures of the radiation sensor. The image may be captured at the full bit depth of the image sensor.

The image may then be shifted up in bit space (that is, a binary bit shift is performed) to match the number of images collected in the dark image sum (for example, if 16 images were calculated to device the averaged dark image, then the image may be shifted into 14-bit space).

At step 420 the received image data is compared with fixed pattern noise data to produce a corrected image, the fixed pattern noise data derived from an average of a plurality of dark noise images collected using the detection device. The averaged dark image (the fixed pattern noise data) in this example is subtracted from the captured image data. That is, the pixel values of the averaged dark image are subtracted from the corresponding pixel values of the received image data to produce a corrected image. A small image offset may be added to the pixel values of each pixel of the corrected image. For example, with a 10-bit image space, a value that may be chosen is 200 (i.e. 19.5%), or 3200 in 14-bit image space.

At step 430, defective pixels are corrected for. A bad-pixel correction kernel, based on a bad pixel map such as that described above, may be applied to select a pixel value for the centre pixel of the kernel based upon the nearest neighbour values. This value may be the average of the pixel values of the neighbouring pixels. In this way, any pixels of the radiation sensor that are designated as "bad" may be corrected for using the kernel.

At step 440, the pixel values of pixels of the corrected image are compared with a threshold value to produce a binary image. For example, a threshold value may be chosen (this may be the same for each pixel of the radiation sensor or may vary across pixels of the sensor), and for each pixel, if that pixel value is above the threshold a corresponding entry of a binary pixel map is updated to a 1; otherwise, the value is a 0 (or vice versa). Thus a "binary image" is formed, wherein each pixel of the binary image is representative of whether the pixel value of a corresponding pixel of the corrected image is above or below the threshold value.

At step 450, the number of charged particles detected by the radiation sensor are counted. The pixels of the pixel array provided by the radiation sensor may be of a size such that, in response to interaction with received charged particles from the radiopharmaceutical, the created interaction charge carriers in the depletion layer are detectable across multiple pixels. The number of charged particles detected may then be counted by determining, for a pixel of the binary image, whether any neighbouring pixels of the binary image take the same binary value as that pixel. A mask may be used to prevent counting the same pixel twice.

In this way, a determination may be made as to how many, if any, charged particles are detected in the captured image data.

Figure 5 is a block diagram of a computing device 500, such as may be used to receive signals from the laparoscopic probe 200 or the imaging apparatus 300 or the handheld detection device described herein. Other architectures to that shown in Figure 5 may be used as will be appreciated by the skilled person. In some embodiments, the computing device may be integral to the laparoscopic probe or the specimen imaging apparatus or the handheld detection device. In some embodiments, the computing device 500 may be remote from the detection device. In some embodiments, the computing device may in fact comprise a computing system, which may be a distributed computing system.

Referring to the figure, the computing device / controller 500 includes a number of user interfaces including visualising means such as a visual display 510 and a virtual or dedicated user input device 512. The computing device 500 includes a processor 514, a memory 516 and a power system 518.

The computing device 500 comprises a communications module 520 for sending and receiving communications between processor 514 and remote systems. For example, communications module 520 may be used to send and receive communications via a network such as the Internet. Communications module 520 may receive communications from a laparoscopic probe 200 or an imaging apparatus 300.

The computing device 500 comprises a port 522 for receiving, for example, a non-transitory computer readable medium containing instruction to be processed by the processor 514.

The processor 514 is configured to receive data, access the memory 516, and to act upon instructions received either from said memory 516, from communications module 520 or from user input device 512. The processor 514 may be configured to receive a detection signal from the radiation sensor of the detection device, the detection signal representative of interaction charge carriers being created in the depletion layer of the semiconductor of the plurality MOS components providing a pixel array. The processor 514 may be configured to determine whether the detection signal is indicative of detection events at multiple neighbouring pixels of the pixel array. The processor may be configured, if a determination is made that the detection signal is indicative of detection events at multiple neighbouring pixels of the pixel array, to determine that the radiation sensor has received at least one or more charged particles.

The skilled person would appreciate that one or more of the components of the computing device 500 may be integrated with the detection device. The computing device may be fully integrated with the detection device (for example, as part of the specimen imaging apparatus 300). The computing device 500 may be remote from the detection device.

Variations of the described embodiments are envisaged, for example, the features of all the described embodiments may be combined in any way.

The subject may be a human (or human tissue) or may be an animal (or animal tissue). The systems, devices and methods are suitable for use in medicine and other industries, such as veterinary medicine.

The person skilled in the art would appreciate that the methods apparatuses, devices and systems described herein may be used to detect any suitable radiopharmaceutical.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present invention. Accordingly, embodiments provide a program comprising code for implementing a system or method as claimed in any preceding claim and a machine-readable storage storing such a program. Still further, embodiments of the present invention may be conveyed electronically via any medium such as a communication signal carried over a wired or wireless connection and embodiments suitably encompass the same.

The above embodiments have been described by way of example only, and the described embodiments are to be considered in all respects only as illustrative and not restrictive. It will be appreciated that variations of the described embodiments may be made without departing from the scope of the invention which is indicated by the appended claims rather than by the foregoing description.

## Claims

1. A laparoscopic probe for detecting radiation from a radiopharmaceutical administered to a subject, the laparoscopic probe comprising a detection device, the detection device comprising:
a radiation sensor having a plurality of metal-oxide-semiconductor, MOS, components providing a pixel array, a semiconductor of the MOS components configured for interaction charge carriers to be created in the depletion layer of the semiconductor in response to direct interaction with received charged particles emitted from the radiopharmaceutical; and
a light sealing covering, wherein the light sealing covering prevents light from impinging on the pixel array.

2. A laparoscopic probe according to claim 1, wherein the laparoscopic probe further comprises:
a collimator, the collimator to filter out charged particles that impinge upon the collimator at an angle above a threshold angle of incidence, thereby to cooperate with the light sealing covering and the radiation sensor to enable detection by the radiation sensor of a radiation imaging effect.

3. A laparoscopic probe according to any preceding claim, wherein the radiation sensor comprises an image sensor.

4. A laparoscopic probe according to any of claims 2 to 3, wherein the laparoscopic probe is operable in a first mode, in which the laparoscopic probe is configured to enable detection by the radiation sensor of a radiation imaging effect, and wherein the laparoscopic probe is operable in a second mode, in which the laparoscopic probe is configured to enable detection by the radiation sensor of the presence of charged particles.

5. A laparoscopic probe according to any preceding claim, wherein the charged particles from the radiopharmaceutical comprise electrons or positrons.

6. A laparoscopic probe according to claim 5, wherein the radiation emitted from the radiopharmaceutical comprises beta radiation.

7. A laparoscopic probe according to any preceding claim, wherein the pixels of the pixel array are of a size such that, in response to interaction with received charged particles from the radiopharmaceutical, the created interaction charge carriers in the depletion layer are detectable across multiple pixels.

8. A laparoscopic probe according to any preceding claim, the laparoscopic probe further comprising:
a gamma radiation detector configured to detect gamma radiation.

9. A laparoscopic probe according to claim 8, wherein the laparoscopic probe is configured to be switchable between a first mode, in which the laparoscopic probe is configured to detect charged particles, and a second mode, in which the laparoscopic probe is configured to use the gamma radiation detector to detect gamma radiation.

10. A laparoscopic probe according to any preceding claim, further comprising:
a computing device comprising a processor for processing detection events and for signalling a detection to a user, wherein the processor is configured to distinguish detection events resulting from charged particles from detection events resulting from gamma radiation.

11. A laparoscopic probe according to claim 10, wherein, to distinguish detection events resulting from charged particles from detection events resulting from gamma radiation, the processor is configured to:
receive a signal from the radiation sensor, the signal representative of interaction charge carriers being created in the depletion layer of the semiconductor of the plurality MOS components providing a pixel array;
determine whether the signal is indicative of detection events at multiple neighbouring pixels of the pixel array; and
if a determination is made that the signal is indicative of detection events at multiple neighbouring pixels of the pixel array, determine that the radiation sensor has received at least one charged particle.

12. A laparoscopic probe according to claim 11, wherein the processor is further configured to:
if a determination is made that that signal is not indicative of detection events at multiple neighbouring pixels of the pixel array, determine that the radiation sensor has received gamma radiation, and discard detection events resulting from gamma radiation.

13. A method of operating a laparoscopic probe according to any of claims 1 to 12, the method comprising:
receiving a detection signal from the radiation sensor of the laparoscopic probe, the detection signal representative of interaction charge carriers being created in the depletion layer of the semiconductor of the plurality MOS components providing a pixel array;
determining whether the detection signal is indicative of detection events at multiple neighbouring pixels of the pixel array; and
if a determination is made that the detection signal is indicative of detection events at multiple neighbouring pixels of the pixel array, determining that the radiation sensor has received at least one or more charged particles.

14. A method according to claim 13, wherein the radiation sensor comprises an image sensor and wherein receiving a detection signal from the radiation sensor comprises receiving image data from the image sensor, the image data representative of a radiation imaging effect; and, optionally
wherein determining whether the detection signal is indicative of detection events at multiple neighbouring pixels of the pixel array comprises:
comparing the received image data with fixed pattern noise data to produce a corrected image, the fixed pattern noise data derived from an average of a plurality of dark noise images collected using the detection device;
comparing pixel values of pixels of the corrected image with a threshold value to produce a binary image, wherein the pixel value of each pixel of the binary image is representative of whether the pixel value of a corresponding pixel of the corrected image is above the threshold value;
for at least one pixel of the binary image, determining how many adjacent pixels have the same value as the pixel.

15. The laparoscopic probe of any of claims 1-12 further comprising a computer-readable medium having executable instructions thereon which, when executed by a processor, cause the processor to carry out the method of any of claims 13 and 14.

## Patentansprüche

1. Laparoskopische Sonde zum Erfassen von Strahlung von einem Radiopharmazeutikum, das einem Patienten verabreicht wird, wobei die laparoskopische Sonde eine Erfassungsvorrichtung umfasst, wobei die Erfassungsvorrichtung Folgendes umfasst:
einen Strahlungssensor, der eine Vielzahl von Metall-Oxid-Halbleiter-, MOS-Komponenten aufweist, die ein Pixelarray bereitstellen, wobei ein Halbleiter der MOS-Komponenten konfiguriert ist, dass Wechselwirkungsladungsträger in der Depletionsschicht des Halbleiters als Reaktion auf direkte Wechselwirkung mit empfangenen Ladungspartikeln erzeugt werden, die von dem Radiopharmazeutikum emittiert werden; und
eine Lichtabdichtungsabdeckung, wobei die Lichtabdichtungsabdeckung verhindert, dass Licht auf das Pixelarray auftrifft.

2. Laparoskopische Sonde nach Anspruch 1, wobei die laparoskopische Sonde ferner Folgendes umfasst:
einen Kollimator, wobei der Kollimator geladene Partikel herausfiltert, die auf den Kollimator in einem Winkel über einem Schwelleneinfallswinkel auftreffen, um dadurch mit der Lichtabdichtungsabdeckung und dem Strahlungssensor zu kooperieren, um Erfassung eines Strahlungsabbildungseffekts durch den Strahlungssensor zu ermöglichen.

3. Laparoskopische Sonde nach einem vorhergehenden Anspruch, wobei der Strahlungssensor einen Bildsensor umfasst.

4. Laparoskopische Sonde nach einem der Ansprüche 2 bis 3, wobei die laparoskopische Sonde in einem ersten Modus betreibbar ist, in dem die laparoskopische Sonde konfiguriert ist, um Erfassung eines Strahlungsabbildungseffekts durch den Strahlungssensor zu ermöglichen, und wobei die laparoskopische Sonde in einem zweiten Modus betreibbar ist, in dem die laparoskopische Sonde konfiguriert ist, um Erfassung des Vorhandenseins von geladenen Partikeln durch den Strahlungssensor zu ermöglichen.

5. Laparoskopische Sonde nach einem vorhergehenden Anspruch, wobei die geladenen Partikel aus dem Radiopharmazeutikum Elektronen oder Positronen umfassen.

6. Laparoskopische Sonde nach Anspruch 5, wobei die Strahlung, die von dem Radiopharmazeutikum emittiert wird, Betastrahlung umfasst.

7. Laparoskopische Sonde nach einem vorhergehenden Anspruch, wobei die Pixel des Pixelarrays von einer solchen Größe sind, dass als Reaktion auf Wechselwirkung mit empfangenen geladenen Partikeln aus dem Radiopharmazeutikum die erzeugten Wechselwirkungsladungsträger in der Deplationsschicht über mehrere Pixel erfassbar sind.

8. Laparoskopische Sonde nach einem vorhergehenden Anspruch, wobei die laparoskopische Sonde ferner Folgendes umfasst:
einen Gammastrahlungsdetektor, der konfiguriert ist, um Gammastrahlung zu erfassen.

9. Laparoskopische Sonde nach Anspruch 8, wobei die laparoskopische Sonde konfiguriert ist, um zwischen einem ersten Modus, in dem die laparoskopische Sonde konfiguriert ist, um geladene Partikel zu erfassen, und einem zweiten Modus schaltbar ist, in dem die laparoskopische Sonde konfiguriert ist, um den Gammastrahlungsdetektor zu verwenden, um Gammastrahlung zu erfassen.

10. Laparoskopische Sonde nach einem vorhergehenden Anspruch, ferner umfassend:
eine Rechenvorrichtung, die einen Prozessor zum Verarbeiten von Erfassungsereignissen und zum Signalisieren einer Erfassung an einen Benutzer umfasst, wobei der Prozessor konfiguriert ist, um Erfassungsereignisse, die aus geladenen Partikeln resultieren, von Erfassungsereignissen zu unterscheiden, die aus Gammastrahlung resultieren.

11. Laparoskopische Sonde nach Anspruch 10, wobei, um Erfassungsereignisse, die aus geladenen Partikeln resultieren, von Erfassungsereignissen zu unterscheiden, die aus Gammastrahlung resultieren, der Prozessor für Folgendes konfiguriert ist:
Empfangen eines Signals von dem Strahlungssensor, wobei das Signal Wechselwirkungsladungsträger darstellt, die in der Depletionsschicht des Halbleiters der Vielzahl MOS-Komponenten erzeugt werden, die ein Pixelarray bereitstellen;
Bestimmen, ob das Signal Erfassungsereignisse an mehreren benachbarten Pixeln des Pixelarrays angibt; und
wenn eine Bestimmung gemacht wird, dass das Signal Erfassungsereignisse an mehreren benachbarten Pixeln des Pixelarrays angibt, Bestimmen, dass der Strahlungssensor zumindest ein geladenes Partikel empfangen hat.

12. Laparoskopische Sonde nach Anspruch 11, wobei der Prozessor ferner für Folgendes konfiguriert ist:
wenn eine Bestimmung gemacht wird, dass dieses Signal keine Erfassungsereignisse an mehreren benachbarten Pixeln des Pixelarrays angibt, Bestimmen, dass der Strahlungssensor Gammastrahlung empfangen hat, und Verwerfen von Erfassungsereignissen, die aus Gammastrahlung resultieren.

13. Verfahren zum Betreiben einer laparoskopischen Sonde nach einem der Ansprüche 1 bis 12, wobei das Verfahren Folgendes umfasst:
Empfangen eines Erfassungssignals von dem Strahlungssensor der laparoskopischen Sonde, wobei das Erfassungssignal Wechselwirkungsladungsträger darstellt, die in der Depletionsschicht des Halbleiters der Vielzahl MOS-Komponenten erzeugt werden, die ein Pixelarray bereitstellen;
Bestimmen, ob das Erfassungssignal Erfassungsereignisse an mehreren benachbarten Pixeln des Pixelarrays angibt; und
wenn eine Bestimmung gemacht wird, dass das Erfassungssignal Erfassungsereignisse an mehreren benachbarten Pixeln des Pixelarrays angibt, Bestimmen, dass der Strahlungssensor zumindest ein oder mehrere geladene Partikel empfangen hat.

14. Verfahren nach Anspruch 13, wobei der Strahlungssensor einen Bildsensor umfasst und wobei das Empfangen eines Erfassungssignals von dem Strahlungssensor das Empfangen von Bilddaten von dem Bildsensor umfasst, wobei die Bilddaten einen Strahlungsabbildungseffekt darstellen; und optional
wobei das Bestimmen, ob das Erfassungssignal Erfassungsereignisse an mehreren benachbarten Pixeln des Pixelarrays angibt, Folgendes umfasst:
Vergleichen der empfangenen Bilddaten mit Rauschdaten mit festem Muster, um ein korrigiertes Bild zu produzieren, wobei die Rauschdaten mit festem Muster aus einem Durchschnitt einer Vielzahl von Bildern mit dunklem Rauschen abgeleitet sind, die unter Verwendung der Erfassungsvorrichtung gesammelt werden;
Vergleichen von Pixelwerten von Pixeln des korrigierten Bildes mit einem Schwellenwert, um ein Binärbild zu produzieren, wobei der Pixelwert jedes Pixels des Binärbildes darstellt, ob der Pixelwert eines entsprechenden Pixels des korrigierten Bildes über dem Schwellenwert ist;
für zumindest ein Pixel des Binärbildes, Bestimmen, wie viele benachbarte Pixel den gleichen Wert wie das Pixel aufweisen.

15. Laparoskopische Sonde nach einem der Ansprüche 1-12, ferner umfassend ein computerlesbares Medium mit ausführbaren Anweisungen darauf, die, wenn sie durch einen Prozessor ausgeführt werden, den Prozessor veranlassen, das Verfahren nach einem der Ansprüche 13 und 14 durchzuführen.

## Revendications

1. Sonde laparoscopique destinée à détecter le rayonnement en provenance d'un produit radiopharmaceutique administré à un sujet, la sonde laparoscopique comprenant un dispositif de détection, le dispositif de détection comprenant :
un capteur de rayonnement possédant une pluralité de composants métal-oxyde-semi-conducteur, MOS, fournissant un réseau de pixels, un semi-conducteur des composants MOS étant configuré pour que des porteurs de charge d'interaction soient créés dans la couche d'appauvrissement du semi-conducteur en réponse à une interaction directe avec les particules chargées reçues émises par le produit radiopharmaceutique ; et
un revêtement d'étanchéité à la lumière, ledit revêtement d'étanchéité à la lumière empêchant la lumière de heurter le réseau de pixels.

2. Sonde laparoscopique selon la revendication 1, ladite sonde laparoscopique comprenant en outre :
un collimateur, le collimateur pour filtrer des particules chargées qui heurtent le collimateur à un angle supérieur à un angle d'incidence seuil, pour ainsi coopérer avec le revêtement d'étanchéité à la lumière et le capteur de rayonnement pour permettre la détection par le capteur de rayonnement d'un effet d'imagerie de rayonnement.

3. Sonde laparoscopique selon une quelconque revendication précédente, ledit capteur de rayonnement comprenant un capteur d'image.

4. Sonde laparoscopique selon l'une quelconque des revendications 2 à 3, ladite sonde laparoscopique pouvant fonctionner dans un premier mode, ladite sonde laparoscopique étant configurée pour permettre la détection par le capteur de rayonnement d'un effet d'imagerie par rayonnement, et ladite sonde laparoscopique pouvant fonctionner dans un second mode, ladite sonde laparoscopique étant configurée pour permettre la détection par le capteur de rayonnement de la présence de particules chargées.

5. Sonde laparoscopique selon une quelconque revendication précédente, lesdites particules chargées provenant du produit radiopharmaceutique comprenant des électrons ou des positrons.

6. Sonde laparoscopique selon la revendication 5, ledit rayonnement émis par le produit radiopharmaceutique comprenant un rayonnement bêta.

7. Sonde laparoscopique selon une quelconque revendication précédente, lesdits pixels du réseau de pixels possédant une taille de sorte que, en réponse à l'interaction avec les particules chargées reçues en provenance du produit radiopharmaceutique, les porteurs de charge d'interaction créés dans la couche d'appauvrissement soient détectables sur de multiples pixels.

8. Sonde laparoscopique selon une quelconque revendication précédente, la sonde laparoscopique comprenant en outre :
un détecteur de rayonnement gamma configuré pour détecter le rayonnement gamma.

9. Sonde laparoscopique selon la revendication 8, ladite sonde laparoscopique étant configurée pour pouvoir être commutée entre un premier mode, dans lequel la sonde laparoscopique est configurée pour détecter des particules chargées, et un second mode, dans lequel la sonde laparoscopique est configurée pour utiliser le détecteur de rayonnement gamma pour détecter le rayonnement gamma.

10. Sonde laparoscopique selon une quelconque revendication précédente, comprenant en outre :
un dispositif informatique comprenant un processeur destiné à traiter des événements de détection et à signaler une détection à un utilisateur, ledit processeur étant configuré pour distinguer des événements de détection résultant de particules chargées d'événements de détection résultant d'un rayonnement gamma.

11. Sonde laparoscopique selon la revendication 10, pour distinguer des événements de détection résultant de particules chargées des événements de détection résultant d'un rayonnement gamma, ledit processeur étant configuré pour :
recevoir un signal en provenance du capteur de rayonnement, le signal étant représentatif de porteurs de charge d'interaction qui sont créés dans la couche d'appauvrissement du semi-conducteur de la pluralité de composants MOS fournissant un réseau de pixels ;
déterminer si le signal indique des événements de détection au niveau de multiples pixels voisins du réseau de pixels ; et
si la détermination que le signal indique des événements de détection au niveau de multiples pixels voisins du réseau de pixels est faite, déterminer que le capteur de rayonnement a reçu au moins une particule chargée.

12. Sonde laparoscopique selon la revendication 11, ledit processeur étant en outre configuré pour :
si la détermination que le signal n'indique pas d'événements de détection au niveau de multiples pixels voisins du réseau de pixels est faite, déterminer que le capteur de rayonnement a reçu un rayonnement gamma et rejeter les événements de détection résultant du rayonnement gamma.

13. Procédé de fonctionnement d'une sonde laparoscopique selon l'une quelconque des revendications 1 à 12, le procédé comprenant :
la réception d'un signal de détection en provenance du capteur de rayonnement de la sonde laparoscopique, le signal de détection étant représentatif des porteurs de charge d'interaction qui sont créés dans la couche d'appauvrissement du semi-conducteur de la pluralité de composants MOS fournissant un réseau de pixels ;
la détermination pour savoir si le signal de détection indique des événements de détection au niveau de multiples pixels voisins du réseau de pixels ; et
si la détermination que le signal de détection indique des événements de détection au niveau de multiples pixels voisins du réseau de pixels est faite, déterminer que le capteur de rayonnement a reçu au moins une ou plusieurs particules chargées.

14. Procédé selon la revendication 13, ledit capteur de rayonnement comprenant un capteur d'image et ladite réception d'un signal de détection en provenance du capteur de rayonnement comprenant la réception de données d'image en provenance du capteur d'image, les données d'image étant représentatives d'un effet d'imagerie de rayonnement ; et, éventuellement
ladite détermination pour savoir si le signal de détection indique des événements de détection au niveau de multiples pixels voisins du réseau de pixels comprenant :
la comparaison des données d'image reçues avec des données de bruit de motif fixe pour produire une image corrigée, les données de bruit de motif fixe étant dérivées d'une moyenne d'une pluralité d'images de bruit sombres collectées à l'aide du dispositif de détection ;
la comparaison des valeurs de pixel de pixels de l'image corrigée avec une valeur seuil pour produire une image binaire, ladite valeur de pixel de chaque pixel de l'image binaire étant représentative de la condition de supériorité ou non de valeur de pixel d'un pixel correspondant de l'image corrigée par rapport à la valeur seuil ;
pour au moins un pixel de l'image binaire, la détermination de combien de pixels adjacents possèdent la même valeur que le pixel.

15. Sonde laparoscopique selon l'une quelconque des revendications 1 à 12, comprenant en outre un support lisible par ordinateur comportant des instructions exécutables sur celui-ci qui, lorsqu'elles sont exécutées par un processeur, amènent le processeur à effectuer le procédé selon l'une quelconque des revendications 13 et 14.
